# EUROPEAN PATENT APPLICATION

(11) **EP 1 306 427 A1**
(43) Date of publication of application: **02.05.2003**
(21) Application number: 01953333.0
(22) Date of filing: 30.07.2001
(51) Int. Cl.: C12N 5/06, C12N 5/14, C12N 15/09

(54) **METHOD OF PROLIFERATING NATURAL KILLER CELLS**

(30) Priority: 31.07.2000 JP 2000230551
(71) Applicant: Riken, Wako-shi, Saitama 351-0198 (JP)
(72) Inventor: OHNO, Tadao, Tsukuba-shi, Ibaraki 305-0074 (JP); HARADA, Hideki, Tsukuba-shi, Ibaraki 305-0074 (JP)
(74) Representative: Sternagel, Fleischer, Godemeyer & Partner Patentanwälte
(86) International application number: JP0106534
(87) International publication number: WO02010350

(57) **Abstract**

An anchorage-dependent cell, which is characterized to be introduced with a detection means, for stimulation of proliferation of a human natural killer cell, and a method for expansion culture of a human natural killer cell by using the aforementioned cell. The cell has a stimulatory action on proliferation of a human natural killer cell and/or a human natural killer precursor cell and is highly sensitive to the human natural killer cell obtained by the expansion culture.

## Description

### Technical Field

The present invention relates to a method for expansion culture of human natural killer cells.

### Background Art

Natural killer (hereinafter also abbreviated as "NK") cells are lymphoid cells which participate in immune reactions. These cells have variety of functions, especially have strong activities for killing tumor cells, and therefore, it is considered that NK cells are one of important members in immunological surveillance mechanism in a living body for removing tumor cells or abnormal cells under tumor progression. For this reason, studies have been made since early days to effectively utilize the cells for tumor therapy and elimination of virus infected cells considered as sources of tumor generation.

The inventors of the present invention found that the anchorage-dependent human Wilms' tumor cell line HFWT was highly sensitive to cytotoxic action of NK cells, like the floating cell line K562. They also found that, when human peripheral blood mononuclear cells (hereinafter sometimes abbreviated as "PBMC") were cultured to proliferate NK cells, the cell line HFWT more strongly stimulated NK cell proliferation than K562 which is well known as a cell line highly sensitive to NK cells (the specification of Japanese Patent Application No. 11-336079). Since PBMC contain NK cells and NK precursor cells, when HFWT cells are used as proliferation-stimulating cells, an NK cell expansion method that is more efficient than conventional methods can be utilized. Further, NK cells, which are basically floating cells, and adhesive HFWT cells can be easily separated by culture medium replacement, and therefore, cell therapy of human tumor by cultured NK cells with a low risk of contamination of survived HFWT cells is becoming possible.

Further, when HFWT cells are used as target cells in measurement of cytotoxic activity of NK cells, survived HFWT cells can be easily separated from floating NK cells since the survived HFWT cells are adhesive. By utilizing this property, the inventors provided a highly safe method for measuring cytotoxic activity of human NK cells based on a method of staining survived HFWT cells with crystal violet for quantification without using a radioactive substance Cr-51 for labeling target cells which has conventionally been used as a standard (Watanabe, S., et al., 2000 World Congress on In Vitro Biology, 6.10.2000, San Diego).

However, when NK cells are selectively cultured from PBMC using HFWT as proliferation-stimulating cells to separate floating NK cells from adhered survived HFWT cells, some of activated NK cells lightly adhere to a culture surface. Therefore, a problem arises that, when these cells are recovered, a part of HFWT cells are most likely to be removed from the culture surface together with the NK cells and floated, and then mix in the NK cells. Since HFWT cells are tumor cells, and considering a use of the cultured NK cells for medical purpose, contamination with living HFWT cells should be most strictly prevented. However, no method has been available to date that enables convenient detection of living HFWT cells mixed in a floating state.

### Disclosure of the Invention

An object of the present invention is to provide a method that enables convenient and highly sensitive detection of tumor proliferation-stimulating cells which may mix in cultured NK cells as described above. Another object of the present invention is to provide a method for producing human NK cells with a very low risk of contamination by survived tumor cells using the aforementioned detection method, and human NK cells obtained by said production method. A further object of the present invention is to provide a method for measuring cytotoxic activity of human NK cells, which is free from use of a radioactive labeling substance so as to be highly safe, and more sensitive compared with the dye staining method.

The inventors of the present invention conducted various studies to achieve the foregoing objects. As a result, they found that, when proliferation-stimulating cells were introduced with a detection means and used for expansion culture of human natural killer cells, proliferation-stimulating cells surviving and mixing in the culture were easily detectable with high sensitivity, and that natural killer cells substantially free from the proliferation-stimulating cells were producible by utilizing said means. The inventors of the present invention also found that proliferation-stimulating cells, introduced with a gene coding for a green fluorescent protein derived from Aequorea as one of the detection means, were conveniently detectable under a fluorescence microscope with extremely high sensitivity, and that, in expansion culture of NK cells from human PBMC using a daughter cell line, efficient culture was achievable by choosing the daughter cell line beforehand which has a high proliferation stimulating ability for NK cells/NK precursor cells in PBMC, and then performing a co-culture by using the chosen cell line as proliferation-stimulating cells for expansion culture of human NK cells. The present invention was achieved on the basis of these findings.

The present invention thus provides anchorage-dependent proliferation-stimulating cells used for expansion culture of human natural killer cells in the presence of the proliferation-stimulating cells, characterized in that said cells are introduced with a detection means. The aforementioned cells have stimulatory action on proliferation of human natural killer cells and/or human natural killer precursor cells so as to be suitable for the expansion culture of human natural killer cells, and are preferably highly sensitive to the human natural killer cells obtained by the expansion culture. Preferred cells are those obtained by introducing the detection means into the human Wilms' tumor cell line HFWT. As the detection means, any means is chosen that enables highly sensitive and convenient detection of the survived cells during proliferation or after culture of the human natural killer cells.

According to preferred embodiments of the present invention, there are provided the aforementioned cells wherein the aforementioned detection means is introduced by a genetic recombination technique. Examples of the genetic recombination technique include, for example, introduction of an exogenous gene, modification of a gene and the like. Introduction of the detection means can be achieved by introducing an exogenous gene that produces, for example, a dye metabolizing enzyme, a fluorescent protein, an antigenic protein, an antibody or the like, or modifying a gene. For example, a means for introducing a gene that produces an Aequorea-derived green fluorescent protein (abbreviated occasionally as "GFP") is preferred. According to another preferred embodiment, there are provided the aforementioned cells wherein the detection means is introduced by a non-genetic recombination technique. Example of the non-genetic recombination technique include introduction of fluorochrome and the like.

Examples of preferred cells provided by the present invention include cell line GHINK-1. Said cell was created by introducing a green fluorescent protein gene into the human Wilms' tumor cell line HFWT by genetic recombination, and therefore, the green fluorescent protein can be used as the detection means. This cell strain was deposited at the International Patent Organism Depositary, the independent administrative corporation, National Institute of Advanced Industrial Science and Technology (Chuo Dai-6, 1-1 Higashi 1-chome, Tsukuba-shi, Ibaraki-ken, Japan) on July 27, 2000 with an accession number of FERM P-17978, and the deposition was transferred to the international deposition under the provisions of the Budapest Treaty on July 17, 2001 (accession number: FERM BP-7668).

From another aspect, the present invention provides a method for expansion culture of human natural killer cells in the presence of proliferation-stimulating cells, characterized in that the proliferation-stimulating cells are anchorage dependent and said cells are introduced with a detection means. In the above method, whether or not the proliferation-stimulating cells are mixing in the human natural killer cells after the proliferation can be easily confirmed by using the aforementioned detection means, and thus human natural killer cells substantially free from survived proliferation-stimulating cells can be produced. In the above method, human natural killer cells can be preferably expanded from human peripheral blood mononuclear cells isolated from human peripheral blood.

From a further aspect, the present invention provides a method for producing human natural killer cells, which comprises the step of subjecting human natural killer cells to expansion culture in the presence of proliferation-stimulating cells, characterized in that the proliferation-stimulating cells are anchorage dependent and introduced with a detection means.

The present invention further provides human natural killer cells obtained by the expansion culture according to the aforementioned method. Preferably, after the aforementioned expansion culture, survived proliferation-stimulating cells can be removed from the culture by using the aforementioned detection means. The human natural killer cells are substantially free from survived proliferation-stimulating cells, and can be safely used for a cell therapy of a human tumor. The present invention provides medicaments for cell therapy of a human tumor, which comprise the aforementioned human natural killer cells, and methods for therapeutic treatment of a human tumor, which comprise the step of administering the aforementioned human natural killer cells to a human tumor patient.

From a still further aspect, the present invention provides methods for measuring cytotoxic activity of human natural killer cells by using the aforementioned proliferation-stimulating cells as target cells. The human natural killer cells, which are obtained by the aforementioned method for proliferating human natural killer cells by using proliferation-stimulating cells and substantially free from the proliferation-stimulating cells, and of which cytotoxic activity is verified by using the proliferation-stimulating cells as target cells, can be most preferably used for cell therapy of a human tumor. The present invention further provides a method for measuring cytotoxic activity of natural killer cells in blood collected from a human individual, in which the aforementioned proliferation-stimulating cells are used as target cells.

### Brief Explanation of Drawings

Fig. 1 shows differences in sensitivity of target cells when cytotoxic activity of NK cells was measured by using the CV staining method.

Fig. 2 shows results of comparison of 4-hour assays of cytotoxic activities of NK cells by the method of measuring fluorescence intensity using the GHINK-1 cells as target cells and the CV staining method.

### Best Mode for Carrying out the Invention

The entire disclosures of Japanese Patent Application No. 2000-230551 (filed on July 31, 2000) are incorporated in the disclosures of the present specification by reference.

The cells provided by the present invention are anchorage-dependent proliferation-stimulating cells used for expansion culture of human natural killer cells in the presence of the proliferation-stimulating cells, characterized in that a detection means is introduced in the cells. The aforementioned cells have a stimulating action on proliferation of human natural killer cells and/or human natural killer precursor cells so as to be suitable for expansion culture of human natural killer cells, and preferably are highly sensitive to the human natural killer cells obtained by the expansion culture. The detection means is preferably selected so as to facilitate easy and highly sensitive detection of the proliferation-stimulating cells surviving during or after the expansion culture of the human natural killer cells. The method for producing human natural killer cells of the present invention is characterized by using the aforementioned proliferation-stimulating cells.

The detection means can be introduced into the cells by a genetic recombination technique or a non-genetic recombination technique. Examples of the genetic recombination technique include, for example, introduction of an exogenous gene, gene modification and the like. Introduction of the detection means can be performed by introduction of an exogenous gene that produces, for example, a dye metabolizing enzyme, a fluorescent protein, an antigenic protein, an antibody or the like, or by gene modification. For example, a means for introducing a gene that produces an Aequorea-derived green fluorescence protein is preferred. For the genetic recombination, a cell that is modified so as not to produce a specific gene product by a knock out of the specific gene in the cell can also be utilized. Example of the non-genetic recombination technique include introduction of fluorochrome and the like.

The aforementioned proliferation-stimulating cells can be generally obtained by (1) a method comprising:
(a) the step of modifying cells by a genetic recombination technique, which are selected from the group consisting of anchorage-dependent cells having stimulatory action on proliferation of human NK cells and/or NK precursor cells and anchorage-dependent cells having sensitivity to cytotoxic action of human natural killer cells, so that surviving cells thereof are easily detectable with high sensitivity; and
(b) the step of selecting anchorage-dependent cells having stimulatory action on proliferation of human NK cells and/or NK precursor cells from the cell line obtained in the above step (a).

When a detection means is introduced into cells by applying a non-genetic recombination technique, cells having proliferation-stimulating action are selected beforehand and then introduce the detection means into the cells. The proliferation-stimulating cells can be obtained by, for example, (2) a method comprising the step of subjecting cells derived from anchorage-dependent cells having stimulatory action on proliferation of human NK cells and/or NK precursor cells to labeling treatment using a fluorochrome or the like to modify the cells so as to be easily detectable with high sensitivity.

Types of the detection means selected for easy and highly sensitive detection of the cells are not particularly limited. Generally, methods known to those skilled in the art can be employed. An examples includes a method of introducing a gene into cells to be manipulated by a genetic recombination technique, wherein said gene can express a gene product in the cells after the introduction, that is easily detectable with high sensitivity, but not produce the product in the cells before the introduction. The proliferation-stimulating cells surviving during or after the culture are easily detectable with high sensitivity by detecting the gene product. Types of the gene are not particularly limited. For example, a gene coding for a fluorescent protein can be used. As the gene coding for a fluorescent protein, for example, an Aequorea-derived GFP gene can be used. Further, for example, β-glucuronidase gene (lac Z) derived from *Escherichia coli* can also be used.

These methods for gene introduction and methods for detection of gene products are well known to those skilled in the art, and those skilled in the art can perform gene introduction and detection of a gene product by choosing an appropriate method or appropriately combining two or more kinds of methods. For example, when a GFP gene is introduced, surviving proliferation-stimulating cells can be easily detected with high sensitivity by observing the cells under a fluorescence microscope. An introduced gene itself that does not exist in a cell before the introduction may be used as a detection object. As convenient methods for detection of an introduced gene, methods well known to those skilled in the art may be used. For example, polymerase chain reaction method, a method using the PCR and a fluorescence detection method in combination (Heid, A.C., et al., Genome Res., 6, 995-1001, 1996), the LAMP method (Notomi, T., et al., Nucleic Acid Res., 28, e63, 2000) and the like may be used.

For detection of surviving proliferation-stimulating cells during the expansion culture of NK cells, floating NK cells are removed and then detection can be performed for adhering cells. For detection of proliferation-stimulating tumor cells mixing in recovered floating NK cells after expansion culture of NK cells, the NK cells can be recovered by a method for separating and recovering viable cells and then detection can be performed for the recovered cells. The method for separating and recovering viable cells is not particularly limited. For example, a method of recovering human lymphocyte fractions by using a commercially available Lymphoprep (Nycomed) can be employed.

Original characteristic properties of subject cells may be lost due to a genetic recombination operation applied to the cells. Therefore, the step of selecting an appropriate daughter cell line from modified daughter cell strains derived from subject cells (the aforementioned step (b) in (1)) may desirably be employed. In the aforementioned step (b), the method for selecting an anchorage-dependent cell line having stimulatory action on proliferation of human NK cells and/or NK precursor cells is not particularly limited. For example, a method can be employed for cloning a cell line in which a stimulatory action on proliferation of NK cells/NK precursor cells is maintained, acquired or enhanced in view of ability of selectively proliferating NK cells from PBMC as an index. As the cloning method, methods well known to those skilled in the art can be used. For example, a daughter cell line can be cloned from objective cells for the selection by using the limiting dilution method. These daughter cells can be proliferated and examined as to whether or nor NK cells selectively proliferate from human PBMC to choose an appropriate daughter cell having a high stimulatory effect on the proliferation.

According to a preferred embodiment of the method of the present invention, natural killer cells can be proliferated from human peripheral blood mononuclear cells isolated from human peripheral blood. The method for examining whether or nor NK cells selectively proliferate from human peripheral blood mononuclear cells is not particularly limited. For example, the method described in Japanese Patent Application No. (Hei)11-336079 can be employed. For the method, daughter cells to be tested can be used instead of HFWT cells.

To introduce a detection means by a non-genetic recombination technique, for example, anchorage-dependent cells having stimulatory action on proliferation of human natural killer cells and/or NK precursor cells can be selected beforehand according to the method described in Japanese Patent Application No. (Hei)11-336079 and the cells can be labeled with a substance for detection. Types of the substance for detection are not particularly limited. For example, fluorochrome PKH26 (Chang, I-K., et al., Cell Biol. Intern., 19, 569-576, 1995) or the like can be used, which has only a weak cytotoxicity and a reduced action of decreasing a cell survival rate. By using a fluorochrome having a reduced cytotoxicity, inhibition of the process of selectively proliferating NK cells from PBMC is prevented to achieve efficient harvest of NK cells. As the method for labeling cells with a fluorochrome, methods known to those skilled in the art can be used. Cells modified by these methods, per se, can be applied to expansion culture of human NK cells/NK precursor cells.

In expansion culture of NK cells using human NK cells/NK precursor cells, methods in which proliferation-stimulating cells are used, per se, have been utilized by those skilled in the art. For example, according to the method described in Japanese Patent Application No. (Hei)11-336079, by using the proliferation-stimulating cells of the present invention instead of HFWT cells and carrying out co-culture, for example, with human PBMC, NK cells in PBMC can be selectively proliferated.

According to the method of the present invention, proliferation ability of the proliferation-stimulating cells need not be essentially lost beforehand for expansion culture of NK cells from human PBMC, which differs from the method described in Japanese Patent Application No. (Hei)11-336079. Even when the proliferation-stimulating cells are proliferated during the expansion culture of NK cells and mixed in finally recovered floating NK cells, they can be easily detected. Moreover, since the contaminating cells are basically adhesive, and accordingly, the cells can be easily eliminated from the cultured NK cells having a floating nature by an operation for adhesion to a culture surface which is well known to those skilled in the art. However, when cultured NK cells are separated from the proliferation-stimulating cells, it is preferred to eliminate proliferation ability of the proliferation-stimulating cells beforehand to reduce a possibility of contamination of the proliferation-stimulating cells as low as possible. The method for eliminating the proliferation ability is not particularly limited. Methods known to those skilled in the art such as radiation exposure and Mytomicin C treatment can be used. The treatment successfully prevents the proliferation-stimulating cells from proliferation during expansion culture of NK cells. In addition, since proliferation-stimulating tumor cells are anchorage dependent, it becomes possible to significantly reduce the possibility of contamination with the proliferation-stimulating cells after the separation of NK cells.

According to the method of the present invention, by culturing PBMC isolated from peripheral blood of a healthy individual, NK cells of the host as a donor of the peripheral blood can be proliferated in a large quantity with maintained high cytotoxic activity. In addition, contamination of proliferation-stimulating cells can be conveniently detected with high sensitivity, and the proliferation-stimulating cells and NK cells can be easily separated. Therefore, NK cells substantially free from proliferation-stimulating cells can be efficiently prepared. PBMC isolated from a tumor patient or an infected patient may also be used, thereby efficient expansion culture of NK cells specific to the patient can be achieved.

Further, by using the proliferation-stimulating cells of the present invention, a level of proliferation-stimulating cells surviving during the expansion culture of NK cells and after the culture can be easily detected with high sensitivity. After cultured NK cells are separated from the proliferation-stimulating cells, the proliferation-stimulating cells contaminated in the NK cells, if any, are easily and highly sensitively detectable. Specific techniques for these methods will be explained in detail in the examples of the specification.

The method for measuring cytotoxic activity of human NK cells of the present invention is characterized to use the aforementioned proliferation-stimulating cells as target cells upon measurement of cytotoxic activity of human NK cells. Since the target cells are anchorage dependent, and since they can be easily separated from NK cells having a floating nature and can be easily and high sensitively detectable, a higher sensitivity is achieved than that of the conventional staining method (the method for measuring cytotoxic activity of NK cells by using HFWT cells as target cells and quantifying surviving HFWT cells with crystal violet staining, described in Japanese Patent Application No. (Hei)11-336079). Furthermore, since no radioactive substance is used, this method can be used as a safe method for measuring cytotoxic activity of NK cells. For example, a level of natural killer activity in blood cells from which NK cells are derived can be measured and used as an index of health state of an individual to be examined. Blood transfusion for a purpose of effective utilization of NK activity for tumor treatment may also become possible.

### Examples

The present invention will be explained more specifically with reference to examples. However, the scope of the present invention is not limited to these examples.

### Example 1: Preparation of a daughter cell line, GHINK-1, of HFWT cells introduced with GFP gene

### (A) Method

(1) Method for introducing GFP gene into HFWT cells
   100 µ1 of a HamAF12 medium not containing fetal bovine serum was added with 3 µl of transfection reagent (Fugene 6, Boehringer Manheim) and left standing at room temperature for 5 minutes. The medium was further added with 2 µg of plasmid vector containing a GFP gene (pEGFP-N1, Clontech) and left standing at room temperature for 15 minutes. This mixture was added to HFWT cells (2 x 10⁵ cells/35 mm plastic dish) which was cultured overnight beforehand.
(2) Cloning of GFP gene-introduced HFWT cells
   Two days after the operation of the above (1), 500 µg/ml of antibiotic G418 was added to kill cells which did not contain a plasmid vector having the resistance gene for the antibiotic. It was observed under a fluorescence microscope that most of the surviving cells expressed GFP. Further, to eliminate a small number of contaminating cells which did not express GFP, the cells were separated into single cells by trypsin treatment, suspended in a medium, and then diluted stepwise and inoculated in a 96-well plate with an average number of 1 cell per well. After cultured for 1 week, each well in which all of proliferated cells expressed GFP were selected under a fluorescence microscope, and subculture was appropriately repeated starting from the wells to increase culture scale. Then, it was verified under a fluorescence microscope and using a flow cytometer that all of the proliferated cells had fluorescence. By the above procedure, 10 daughter cell lines deriving from HFWT cells, which expressed GFP gene products, were obtained. These lines were designated as GHINK-1 to GHINK-10.
(3) Induction culture of NK cells
   Induction culture of NK cells by using PBMC, which is known to contain human NK cells and/or human NK precursor cells, was conducted according to the method described in Japanese Patent Application No. (Hei)11-336079. Briefly, the cells of 10 strains of GHINK series cultured overnight beforehand (1 x 10⁵ cells per well) were irradiated with X-ray, and added with 1 x 10⁶ cells per well of PBMC derived from a healthy individual or cord blood. A medium for induction culture of NK cells was added with 200 U/ml of interleukin-2 (IL-2), and the cells were cultured at 37°C in a CO₂ incubator.
(4) Flow cytometry
   The lymphocytes cultured in the above (3) were stained with fluorescence on days 6 to 10 of the culture by using fluorescence labeled monoclonal antibodies specifically binding to CD3 which is a surface marker of a T cell, and to CD56 which is a surface marker of a NK cell. After the staining, a proportion of NK cells, which are CD56-positive and CD3-negative cells, was measured by the flow cytometry method well known to those skilled in the art.
(5) Count and measurement of relative fluorescence intensity of GHINK series cells
   To examine whether or not proliferation property of the GHINK series cells was degraded by the introduction of the GFP gene, 1 x 10⁴ of the GHINK-series cells or the parent HFWT cells were inoculated on a 96-well plate. The cells were dispersed by trypsin treatment on day 2, 6 and 9 of the culture, and the number of cells was counted by using a Tatai hemocytometer to obtain the number of cells per ml. Further, fluorescence intensity of each well was measured by a fluorometer for a 96-well plate (a fluorescence plate reader) before the counting, and for convenience, a value obtained by dividing the intensity with the number of cells in each well was regarded as relative fluorescence intensity per cell.

### (B) Results

The proportion (%) of NK cells (CD56-positive and CD3-negative cells) obtained by the induction culture using PBMC derived from the cord blood and the total cell number per well are shown in Table 1, wherein the 10 strains of GHINK series obtained by the aforementioned method were used as cells for stimulation of NK cell proliferation.

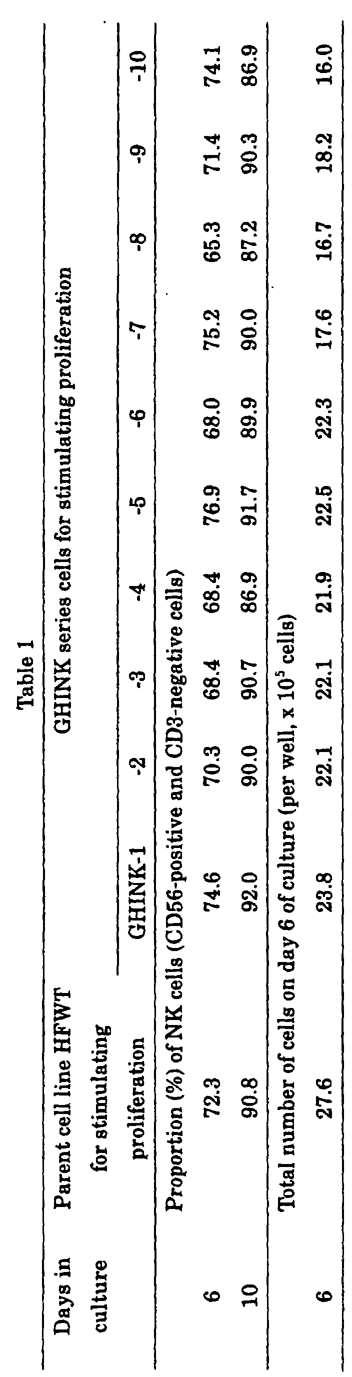

The results of the above experiment indicate that, when the GHINK-1 cells were used as target cells, the proportion of NK cells induced and proliferated from PBMC reached to 74.6% on day 6 of the culture, and the total number of cells per well also increased to 23.8 x 10⁵ cells, which was the largest among the daughter cell lines although proliferation was slower than the parent HFWT cell strain. In addition, it was observed that the proportion of NK cells reached to the maximum level of 92.0% on day 10 of the culture, which reveals a superior stimulatory effect on the NK cell proliferation. It was also recognized under a fluorescence microscope that, with a progress of proliferation of the NK cells, remaining GHINK-1 cells was disappearing with the increase of the number of lymphocytes. The results suggest that whether or not proliferation stimulating tumor cells remain after induction of NK cells can be easily detected by fluorescence observation.

Generally, proliferation property of cells introduced with an exogenous gene may sometimes be degraded. However, in the above examples, no significant degradation of proliferation ability was observed among the daughter cell lines except for the GHINK-5 cells. A cell suspension was prepared so as to have cell number of 1 x 10⁵ cells/ml immediately after the start of the culture, and then 1 ml of the suspension was uniformly inoculated in each well. As a result, the cell numbers of the daughter cell lines on day 2 of the culture rather decreased to 1 x 10⁵ cells/ml or less, except for GHINK-1, GHINK-6 and GHINK-9. In comparison with the weak adhesion property of the parent HFWT cell line, GHINK-1, GHINK-6 and GHINK-9 cell lines had relatively higher adhesion property among the cloned GFP-introduced cell lines. Accordingly, these cell lines had a characteristic feature that they were easily separated from NK cells proliferated from PBMC. Further, relative fluorescence intensity per cell was compared among the cell lines. The results are shown in Table 2.

**Table 2**

| Parent cell line | GHINK series cells | | | | | | | | | |
|---|---|---|---|---|---|---|---|---|---|---|
| Cell line | | | | | | | | | | |
| HFWT | GHINK-1 | -2 | -3 | -4 | -5 | -6 | -7 | -8 | -9 | -10 |

| Relative fluorescence intensity | | | | | | | | | | |
|---|---|---|---|---|---|---|---|---|---|---|
| 0 | 1.54 | 0.57 | 0.46 | 0.35 | 0.63 | 0.51 | 0.82 | 0.29 | 0.82 | 0.36 |

As clearly shown in Table 2, GHINK-1 cell line had the highest relative fluorescence intensity per cell, which was about 2 to 5 times higher relative fluorescence intensity than those of the other GHINK-series cell lines. A higher fluorescence intensity per cell provides higher detection sensitivity. Accordingly, it is apparent that this property is advantageous for detection of surviving proliferation-stimulating cells.

### Example 2: Comparison of CV staining method and fluorometry method of target cells in measurement of cytotoxic activity of NK cells

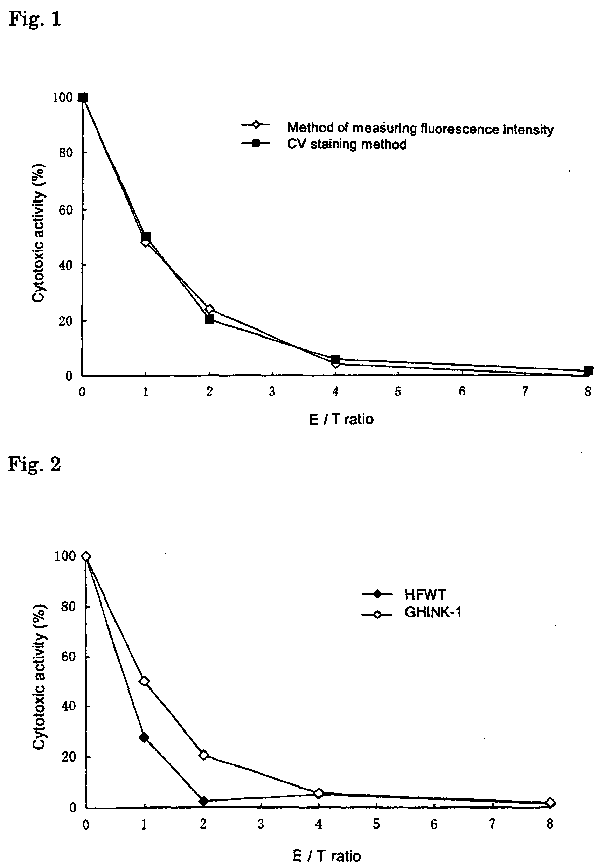

In the method described in Japanese Patent Application No. (Hei)11-336079, the inventors of the present invention used, for the measurement of target cytotoxic activity of NK cell, the crystal violet staining method (hereinafter referred to as CV staining method) and quantified target cells which were not killed and remained. It was revealed that, among the 10 GHINK series cell lines obtained in the above Example 1, the GHINK-1 cell line was the most useful cell line as cells for stimulation of NK cell proliferation. In this experiment, NK cytotoxic activity was measured by utilizing fluorescence emitted from the GHINK-1 cells themselves, and the results were compared with those obtained by the CV staining method.
(A) Method
   (1) Measurement of cytotoxic activity
      Cytotoxic activity of lymphocytes induced and cultured from PBMC was measured by using HFWT cells as proliferation-stimulating cells according to the method described in Japanese Patent Application No. (Hei)11-336079. Briefly, 1 x 10⁴ GHINK-1 cells or HFWT cells were inoculated in each well of a 96-well plate and cultured overnight. The resulting culture was added with lymphocytes containing NK cells obtained beforehand by induction culture from PBMC in a number of 0, 1, 2, 4 or 8 x 10⁴ cells per well. The ratio of the effecter cells (lymphocytes) and the target cells (GHINK-1 cells or HFWT cells) was shown as an E/T ratio, i.e., the E/T ratio was 0, 1, 2, 4 or 8. After culture for 4 hours (hereinafter referred to as "4-hour assay"), each well was washed once with calcium/magnesium free Dulbecco's phosphate buffered saline (hereinafter abbreviated as PBS(-)), and target cells that adhered to and remained on the culture surface as not being killed were stained by the CV staining method to obtain cytotoxic activity.
      In a cytotoxicity test in which only the GHINK-1 cells were used as target cells, a culture supernatant was removed after the 4-hour assay, the well was washed once with PBS(-), and fluorescence intensity of target cells, that adhered to and remained on the culture surface as not being killed, was measured. That is, these target cells were uniformly dissolved in a solution obtained by dissolving a given amount of 5% sodium dodecylsulfate in PBS(-), and fluorescence intensity of each well was measured by using a fluorescence plate reader to obtain cytotoxic activity. For the 4-hour assay, proliferation of the target cells themselves during this period can be ignored, and therefore a value obtained from control target cells not added with the lymphocytes after the 4-hour culture was defined as 100% and used for calculation. The results are shown in Fig. 1.
      When qualification was performed by the CV staining method, the E/T ratio was about 0.5 in the 4-hour assay, that represents a cytotoxic activity value of 50% of the lymphocytes containing NK cells relative to the parent strain HFWT cells, whereas the ratio for the GHINK-1 cells was about 1. Therefore, the GHINK-1 cells were slightly less sensitive than the NK cells. However, it is considered that the difference in sensitivity in this level does not cause any problem in practical measurement of cytotoxic activity. Further, cytotoxic activity of NK cells was measured by each of the method based on measurement of fluorescence intensity and that based on CV staining property by using the GHINK-1 cells, and quantification performances of the GFP fluorescence intensity measurement and the CV staining were compared. The results are shown in Fig. 2.
      Substantially no difference in the E/T ratio was observed between the fluorescence intensity measurement method and the CV staining method in the 4-hour assay, and they gave high data compatibility as for the quantification performance. Therefore, it was revealed that measurement of the GFP fluorescence intensity successfully gave cytotoxic activity of NK cells. In the CV staining method having conveniently been employed by the inventors of the present invention, an absorbance value obtained in a control of only lymphocytes must be subtracted from an absorbance value obtained in remaining target cells, because NK cells that adhered to a culture surface in a slight amount were unselectively stained as well as the target cells. Thus, this method sometimes gave an experimental error in which cytotoxic activity was indicated as 0% or lower. Whilst in the method of the present invention, by performing measurement of cytotoxic activity based on the GFP fluorescence intensity, only the target cells can be specifically measured, because the fluorescent protein is expressed only in the target cells and no fluorescent substance exists in lymphocytes, thereby the method will give higher accuracy compared with the conventional CV staining method.

### Example 3: Measurement of cytotoxic activity of NK cells by fluorometry of released substance by using GHINK-1 cells as target cells

In Example 2 described above, target cells that were not killed by NK cells and survived were quantified. When the E/T ratio is low, only a small portion of cells relative to the whole target cells were killed. Accordingly, a proportion of remaining target cells becomes overwhelmingly large, which may results in frequent quantification errors. To overcome the problem, a method utilizing measurement of fluorescence of GFP released from killed target cells was developed.
(A) Method
   (1) Measurement of cytotoxic activity

Cytotoxic activity of lymphocytes obtained by induction culture from PBMC by using HFWT cells as proliferation-stimulating cells was measured according to the method described in Japanese Patent Application No. (Hei)11-336079. Briefly, 5 x 10⁴ of GHINK-1 cells were inoculated in each well of a 96-well plate and cultured overnight. Then, the culture medium was replaced with 200 µl of MEM medium not containing phenol red but containing 10% fetal bovine serum. This medium was added with the lymphocytes containing NK cells obtained beforehand by induction culture from PBMC in a number of 0, 1, 2, 4 or 8 x 10⁴ cells per well. The ratio of effecter cells (lymphocytes) and target cells (GHINK-1 cells) was represented as an E/T ratio. That is, the E/T ratio was 0, 1, 2, 4 or 8. After culture of 4 hours, 100 µl of culture supernatant was collected from each well and transferred to wells of another microplate. Fluorescence intensity of each well was measured by using a fluorescence plate reader to obtain cytotoxic activity. Cytotoxic activity was represented by the fluorescence intensity obtained by the fluorescence plate reader. The results are shown in Table 3.

**Table 3**

| E/T ratio | 0 | 1 | 2 | 4 | 8 |
|---|---|---|---|---|---|
| Fluorescence intensity | 9493 | 15815 | 21597 | 29226 | 38979 |
| Difference in fluorescence intensity | 0 | 6322 | 12104 | 19733 | 29486 |

The value of the difference in fluorescence intensity increased almost linearly up to the E/T ratio of 2, and even when the E/T ratio was as low as 1, a 4-digit value was obtained, which was 67% higher than the value obtained with an E/T ratio of 0. These results revealed that cytotoxic activity of NK cells was successfully quantified with sufficiently high sensitivity even at a lowerer E/T ratio.

### Industrial Applicability

By expansion culture of human NK cells by using the proliferation-stimulating cells of the present invention, the proliferation-stimulating cells surviving and contaminating in the cultured and recovered NK cells can be easily detected, and the proliferation-stimulating cells and the NK cells are readily separated. Therefore, human NK cells that are substantially free from the proliferation-stimulating cells can be efficiently obtained. For example, when NK cells are proliferated by using PBMC of a malignant tumor patient as a raw material, it becomes possible to produce patient's autologous cultured NK cells that are substantially free from surviving proliferation-stimulating cells and use said cells as a medicament for therapeutic treatment of the malignant tumor.

## Claims

1. An anchorage-dependent cell, which is **characterized** to be introduced with a detection means, for stimulation of proliferation of a human natural killer cell.

2. The cell according to claim 1, which has a stimulatory action on proliferation of a human natural killer cell and/or a human natural killer precursor cell and is highly sensitive to the human natural killer cell that is obtained by the expansion culture.

3. The cell according to claim 1 or 2, which is a daughter cell of human Wilms' tumor cell line HFWT introduced with a detection means.

4. The cell according to any one of claims 1 to 3, wherein the detection means is introduced by a genetic recombination technique.

5. The cell according to any one of claims 1 to 4, which is introduced with a gene producing a green fluorescent protein derived from Aequorea.

6. The cell according to claim 1, which is cell strain GHINK-1 (FERM BP-7668).

7. A method for expansion culture of a human natural killer cell in the presence of a cell for stimulation of proliferation, wherein the cell according to any one of claims 1 to 6 is used as the cell for stimulation of proliferation.

8. The method according to claim 7, wherein a human natural killer cell is proliferated from a human peripheral blood mononuclear cell isolated from human peripheral blood.

9. The method according to claim 7 or 8, which further comprises the step of eliminating surviving cells for stimulation of proliferation from the culture by using a detection means.

10. A human natural killer cell, which is obtainable by the method according to any one of claims 7 to 9.

11. A method for measuring cytotoxic activity of a human natural killer cell, wherein the cell for stimulation of proliferation according to any one of claims 1 to 6 is used as a target cell.
